# EUROPEAN PATENT APPLICATION

(11) **EP 4 782 019 A1**
(43) Date of publication of application: **29.07.2026**
(21) Application number: 25153513.4
(22) Date of filing: 23.01.2025
(51) Int. Cl.: A61L 27/50, A61L 27/58, A61L 31/14, B33Y 80/00

(54) **STRUCTURE COMPRISING A FIRST, RAPIDLY DEGRADABLE PART AND A SECOND, SLOWLY OR NON-DEGRADABLE PART**

(71) Applicant: Fundación IMDEA Materiales, 28906 Getafe (Madrid) (ES); Universidad Politécnica de Madrid, 28040 Madrid (ES); Meotec GmbH, 52068 Aachen (DE)
(72) Inventor: Molina Aldareguia, Jon Mikel, 28024 Madrid (ES); Llorca Martínez, Francisco Javier, 28034 Madrid (ES); Patterson, Jennifer, 28014 Madrid (ES); Díaz Lantada, Andrés, 28003 Madrid (ES); Solórzano Requejo, William Gabriel, 28030 Madrid (ES); Dr. Kopp, Alexander, 52066 Aachen (DE); Pöstges, Simon, 52068 Aachen (DE)
(74) Representative: Bungartz, Florian

(57) **Abstract**

A structure comprising a first part (1) made at least partially from a rapidly degradable material and a second part (2) made at least partially from a slowly or non-degradable material, whereas the second part (2) has an elastically deformed form, which is secured by the first part (1) such that an at least partial degradation of the first part (1) will release an elastic shape change of the second part (2).

## Description

### FIELD OF THE INVENTION

The invention relates to a structure composed of two parts, one made from a rapidly degradable material and the other from a slowly or non-degradable material. The structure is useful in various applications, including medical applications.

### BACKGROUND OF THE INVENTION

So-called four-dimensional (4D) printing uses materials and techniques common from three-dimensional (3D) printing (or additive manufacturing technologies in general), sometimes with slight adaptations, and generates components and devices that perform controlled geometrical changes over time. The transformation is usually enabled by special design features, by the use of digital or stimuli-responsive materials, by multi-material printing or even by actively producing plastic deformation upon the part structure controllably (cf. references 1 - 3). This emergent field of 4D printing is being populated with different examples of materials, technologies and shape-changing principles that can lead to design-controlled metamorphoses, necessary for "smart" structures and actuators, and is starting to find applications in health (cf. references 4 - 5), robotics (cf. references 6 - 7), transport (cf. reference 8), architecture (cf. reference 9), and space (cf. reference 8). Basic strategies for promoting geometrical transformations of additively manufactured (referred to as 3D printed) objects, and thus reaching the desired fourth dimension, include: shape changes led by the actuation of inner stresses, shape changes led by controlled elastic or plastic deformation through external stimuli, shape changes led by pneumatic or hydraulic actuation, shape changes enabled by printed mechanisms, shape changes led by shape-memory effects of special polymers and alloys, and shape changes led by multifunctional materials in general (cf. references 1-3).

Regarding the possibilities provided by the printability of stimuli-responsive materials, including polymers (cf. reference 10), alloys (cf. reference 11), and ceramics (cf. reference 12), mechanically active and remarkable shape-changing structures can be achieved that respond in a controlled manner to temperature (cf. references 10, 13 - 14), voltage (cf. references 15 - 16), light (cf. references 17 - 18) and/or humidity (cf. references 19 - 20), among other options. Considering the benefits of multi-material printing for 4D printed components or shape-changing devices, configurations based on different synergic active materials (cf. reference 21) can lead to multi-stimuli responsive systems (cf. reference 22), self-sensing devices (cf. references 23 - 24), step by step metamorphoses or even repeated actuations (cf. reference 25), which are always challenging in 4D printed parts. Among the different triggering stimuli, biodegradation has been employed to some extent (cf. reference 26) and biodegradable materials have been reported for shape-changing actuators (cf. references 27 - 28).

However, degradation- or biodegradation-based shape-changing triggering has not realized its remarkable capabilities for achieving large shape transformations, through single, multiple or progressive actuation steps. The biodegradation of smart materials and structures is normally used in a specific region of the device, normally for drug eluting purposes: the biodegradable material dissolves and encapsulated agents are liberated (cf. reference 26). In other cases (cf. references 27 - 28), biodegradation of the materials is used for sustainability purposes, but not for triggering motion or activating successive or progressive metamorphoses, as proposed in this invention.

More recently, laser powder bed fusion or selective laser sintering has been also employed to manufacture biodegradable alloys, normally processing a single material degrading progressively but without a selective control or biodegradation by design and lacking shape-changing properties. Among detected studies and applications: 3D printing (by laser powder bed fusion or selective laser sintering) of Zn has been reported as biodegradable metal for bone repair applications based on degradable screws (cf. reference 29) and 3D printed Mg has been also described for biodegradable medical devices (cf. reference 30). Mg has been proposed for biodegradable vertebral fixations minimizing stress-shielding (cf. reference 31) and since many years attracted attention in connection with orthopedics and bone repair in general (cf. references 32 - 33). Despite the benefits of additive manufacturing employing biodegradable metals for bone repair and tissue engineering, detected studies only take advantage from the biomimetic design features that additive manufacturing enables and from the biodegradation of metals like Zn and Mg for solutions leading to progressive bone formation that replaces the metallic structure along the healing process. Other printable biodegradable materials for medical applications have been also proposed, mainly hyaluronic acid and hydrogels for bone repair (cf. reference 34).

Regarding other previous studies, the selective laser sintering of smart alloys (from the "nitinol" or nickel-titanium family in quasi-equiatomic proportions) has been described, as well as their design-empowered properties by using metamaterial-designs for enhanced shape-changing (cf. reference 35). These smart alloys employ thermoelastic martensitic transformations triggered by temperature of mechanical stresses. In a study on 4D printed shape-memory polymers and alloys (cf. reference 36) a reference detailing active origami by 4D printing for empowering shape-changing was detected (cf. reference 37), but without explaining ways of controlling shape-changing by design. Devices employing biological materials that degrade to function in response to environmental conditions like light, humidity or temperature have been also described (cf. reference 38).

Overall, single, multiple or progressive actuation steps, in which rapidly degradable regions, with design-controlled features to fine-tune degradation rates and in some cases subject to plastic deformation, enable the controlled release of slowly degradable regions subject to elastic deformation has not been found in the state-of-the-art. Despite leading to slow actuations, the potential of shape-changing actuators triggered by degradation (or biodegradation) for medical applications is clear, if biomedical biodegradable materials are used, because the healing and growth processes of patients that may benefit from evolutive medical devices are also time consuming. Of special interest are bone distraction devices, skin expanders, tissue engineering scaffolds, endoluminal devices and minimally invasive surgical tools.

Other industrial fields may also benefit from shape-changing actuators triggered by selective or differential degradation (or biodegradation). Potentials transcend the biomedical field and may have an impact on areas including architecture, robotics, transport, mechanical and naval engineering in general, aeronautic technologies or space exploration.

### OBJECTIVE OF THE INVENTION

The invention aims to provide a structure, in particular for medical uses, with improved shape change potential.

### SUMMARY OF THE INVENTION

This objective is achieved by a structure as claimed in claim 1. Preferred uses of the structure are claimed in claim 13. Preferred embodiments of the structure and additional uses are subjects of the additional claims and/or are disclosed in the description.

### DETAILS OF THE INVENTION

The present invention relates to a structure that includes a (i.e. at least one) first part made at least partially, preferably completely from a rapidly degradable material and a (i.e. at least one) second part made at least partially, preferably completely from a slowly or non-degradable material. The second part of the structure is designed to have an elastically deformed form which is secured by the first part. This configuration allows for an at least partial degradation of the first part to trigger or release an elastic shape change of the second part.

The relative terms "rapidly degradable" and "slowly degradable" are to be understood as being comparative to each other, i.e. the rapidly degradable material degrades faster than the slowly degradable material in the same (specific) environment. When compared to a non-degradable material, any degradable material is a rapidly degradable material. For example, the rapidly degradable material may degrade faster with a value between 10% and 50%, or between 50% and 100%, or between 100% and 200%, or between 200% and 400%, or between 400% and 600% than the slowly degradable material, whereas 100% means that the rapidly degradable material degrades twice as fast respectively in half the time compared to the slowly degradable material.

Degradable materials are those that can be broken down by biological, chemical and/or physical processes, typically by the interaction of cells, enzymes, microorganisms such as bacteria or fungi, or with media, ions or any other substance initiating a dissolution or loss in integrity of the material.

In the context of this invention the term "degradable" is regarded as being synonymous with the terms "absorbable", "corrodible" and "resorbable".

The first part of the structure is made from a rapidly degradable material. This material can be any type of material that is designed to degrade over a relatively short period of time due to an exposure to certain environmental conditions.

The second part of the structure is made from a slowly or non-degradable material. This material is designed to maintain its structural integrity over a longer or undefined respectively infinite period of time compared to the rapidly degradable material of the first part (while exposed to the same environmental conditions).

The second part of the structure is designed to have an elastically deformed form. This means that the second part is originally formed or shaped in a certain way, but it is then deformed from its original shape due to the application of an external force. This deformation is at least partially, preferably completely elastic in nature, meaning that the second part is capable of at least partially, preferably completely returning to its original shape once the external force is removed.

The first part of the structure secures the elastically deformed form of the second part.

The degradation of the first part triggers an elastic shape change of the second part. As the first part degrades, it loses its ability to secure the elastically deformed form of the second part. This allows the second part to return at least partially to its original shape due to its inherent elastic properties.

In one embodiment of the invention, the first part of the structure is made from a rapidly biodegradable material and/or the second part is made from a slowly or non-biodegradable material. Biodegradable materials are degradable materials that can be broken down by biological processes, typically by the interaction of cells, enzymes, microorganisms such as bacteria or fungi. This allows for the structure to be used advantageously as an implant. In particular, a structure with all materials used being biodegradable eliminates the need for a second surgery to remove the implant.

In the context of this invention the term "biodegradable" is regarded as being synonymous with the terms "bioabsorbable", "biocorrodible" and "bioresorbable".

In an embodiment of the invention, the second part of the structure is secured by the first part in such a way that a degradation of the first part leads to a spontaneous shape change from the initial, elastically deformed form to a final form, in which the second part is not elastically deformed or less elastically deformed than in the initial form. This may be achieved by a design of the first part in such a way that its degradation leads to a weakening which eventually leads to a catastrophic destruction of the first part.

In another embodiment of the invention, the second part of the structure is secured by the first part in such a way that a progressing degradation of the first part leads to a progressing shape change of the second part. This means that as the first part degrades over time, the second part gradually changes its shape. This progressive shape change can be due to the gradual release of the elastic deformation of the second part as the first part degrades. The rate of shape change of the second part can be directly related to the rate of degradation of the first part. This can provide a controlled and predictable change in the shape of the second part over time. This feature can be particularly useful in applications where a gradual release of the shape change of the second part is desirable. For example, in some applications, it may be beneficial for the second part to slowly return to its original shape over a period of time, rather than changing shape suddenly.

In an embodiment of the invention, the progressing shape change of the second part can be either stepwise or continuous.

In a stepwise shape change, the second part changes its shape in distinct steps. Each step corresponds to a specific degree of the shape change of the second part. For example, the second part may maintain its deformed shape until the first part has degraded to a certain extent, at which point the second part changes its shape to a new form. This process can repeat with further degradation of the first part, resulting in a series of discrete shape changes in the second part. This may be achieved by a design of the first part in such a way that its degradation leads to a weakening of different sections in different timespans, whereas a catastrophic destruction of each of the sections of the first part leads to a partial shape change of the second part.

In a continuous shape change, the second part gradually changes its shape in a continuous manner as the first part degrades. This can result in a smooth transition of the second part from its initial deformed shape to its final shape. The rate of shape change can be directly related to the rate of degradation of the first part, providing a predictable and controlled shape change over time. This may be achieved by a design of the first part in such a way that its degradation leads to a constantly shrinking size of the first part, whereas a shrinking size of the first part leaves room for the second part to constantly expand.

In an embodiment of the invention, the first part and the second part of the structure are integral sections of an integrated element. This means that the first and second parts are not separate pieces, but rather, they are different sections of a single, unified element, whereas the different materials merge into each other at a border between the two parts.

This integrated element can be manufactured using a variety of methods, including but not limited to, gluing, welding, fusing, molding, extrusion, or, preferably, additive manufacturing techniques. The use of an integrated element can provide several advantages. For example, it can simplify the manufacturing and assembly process by reducing the number of separate parts that need to be produced and assembled. It can also improve the structural integrity of the structure.

In this embodiment, the rapidly degradable material of the first part and the slowly or non-degradable material of the second part can be incorporated into the integrated element in a variety of ways. For example, they could be arranged in different layers, sections, or regions of the integrated element.

In another embodiment of the invention, the first part and the second part of the structure are separate elements. Thus, the first and second parts are distinct components that are assembled together and thereby connected to each other to form the structure. The separate elements can be manufactured independently using a variety of methods, including but not limited to, gluing, welding, fusing, molding, extrusion, or additive manufacturing techniques. Once manufactured, the first and second parts can be assembled together using a variety of methods, such as mechanical fastening, adhesive bonding, thermal bonding, or other similar methods. Generally, the connection between the parts can be based on a form-fit and/or an adhesive-fit and/or a frictional fit. The use of separate elements for the first and second parts can provide several advantages. For example, it can allow for greater flexibility in the design and manufacturing of each part, as each part can be more easily optimized for its specific function and material properties.

In an embodiment of the invention, both, the first and second parts of the structure each comprise at least one interlocking element. These interlocking elements mechanically interact to create a connection between the first and second parts, securing the second part in its elastically deformed form. The interlocking elements can take a variety of forms, including but not limited to, hooks, tabs, grooves, ridges, staples, rivets or other structures. The specific design of the interlocking elements can be tailored to suit the requirements of the particular application. As the interlocking elements of the first part degrade, they lose their ability to maintain the connection with the interlocking elements of the second part. This leads to a release of the connection between the first and second parts, allowing the second part to return to its original shape due to its inherent elastic properties.

In another embodiment of the invention, the first part of the structure is plastically deformed when the second part has the elastically deformed form. This means that the first part, which is made from a rapidly degradable material, is originally formed or shaped in a certain way and then deformed or distorted from its original shape due to the application of an external force. Unlike elastic deformation, plastic deformation is at least to some extent permanent and the first part does not return to its original shape once the external force or pressure is removed. The plastic deformation of the first part can serve several purposes. In particular, the plastic deformation of the first part may allow to easily bring the second part into its elastically deformed form by deforming both parts simultaneously whereas the deformation of the second part is elastic and the deformation of the first part is plastic, thus securing the elastically deformed form of the second part.

The plastic deformation of the first part and/or the elastic deformation of the second part can be achieved through various methods, including but not limited to, bending, stretching, compressing, compacting or other similar methods.

In an embodiment of the invention, either the first part, the second part, or both parts of the structure are produced by additive manufacturing. Accordingly, the invention is also directed to a method of producing a structure according to the invention by additive manufacturing of either the first part, the second part, or both parts.

Additive manufacturing, also known as 3D printing, is a process of creating three-dimensional objects by building up the object layer by layer or continuously growing the object in the direction of at least one axis, which allows for the creation of complex shapes and structures.

The use of additive manufacturing to produce the first part and/or the second part of the structure can provide several advantages. For example, it can allow for greater design flexibility, as complex shapes and structures can be easily created. It can also allow for more efficient use of materials, as material is only added where it is needed. Furthermore, additive manufacturing can enable rapid prototyping and customization, as changes to the design can be easily made. Further, a structure according to the invention, in which the first part and the second part are integral sections of an integrated element, can be advantageously produced by additive manufacturing, including multi-material approaches, where one or more materials are combined (in a form-fitting and/or adhesive-fitting manner) in one building job.

In general, multi-material additive manufacturing, including material extrusion, material jetting, binder jetting, vat photopolymerization, powder bed fusion, sheet lamination and direct energy deposition, promotes geometrical complexity and facilitates the creation of complex-shaped structures.

In an embodiment of the invention, the rapidly degradable material of the first part and/or the slowly or non-degradable material of the second part can be or comprise at least a metal (pure or as the main component of an alloy) and/or a polymer and/or a ceramic. The specific type of metal, polymer, or ceramic used for the first part or the second part can depend on the desired rate of degradation, the environmental conditions to which the structure will be exposed, and other factors.

As polymers of special interest, in particular for additive manufacturing, there is polyvinyl alcohol, polylactic acid, polycaprolactone, polyethylene terephthalate, polyethylene terephthalate glycol, epoxy resins, methacrylate resins, polyethylene glycol diacrylate, shape-memory polymers, biophotopolymers and biophotoelastomers. Among alloys of special interest there is Mg alloys, Zn alloys, Fe alloys and various families of steels, Ni alloys, Ti alloys and Ni-Ti (nitinol) alloys, while other precious metals like Au, Ag, Cu and Pt may also form part of the structure. As for ceramics, the combined employment of hydroxyapatite, tricalcium phosphate, alumina, zirconia, alumina toughened zirconia, zirconia toughened alumina, silicon nitride, calcium silicate and lithium disilicate can lead to a beneficial structure, in particular manufactured by multi-material lithography-based manufacturing.

Specifically, the rapidly degradable material of the first part can be or comprise at least one of the following: magnesium (Mg), zinc (Zn), titanium (Ti), polyvinyl alcohol (PVA), polycaprolactone (PCL), or hydroxyapatite. For example, magnesium and zinc are metals that can corrode rapidly when exposed to certain environmental conditions. Further, magnesium and zinc are absorbable and can thus be used beneficially for an implant. Polyvinyl alcohol and polycaprolactone are types of biodegradable polymers that can be broken down by biological processes. Hydroxyapatite is a type of bio-ceramic that can be absorbed by the body over time as well.

The slowly or non-degradable material of the second part can be or comprise at least one of the following: zinc (Zn), iron (Fe), titanium (Ti), polyethylene terephthalate (PET), polyetheretherketone (PEEK), polypropylene (PP), polyvinylidenefluoride (PVDF), polyethylene terephthalate glycol (PETG), polylactic acid (PLA), acrylonitrile butadiene styrene (ABS), tricalcium phosphate, alumina, or zirconia.

The use of these specific materials for the first and second parts allows to design different structures within a wide range of functional properties, allowing the structure to be tailored to a wide range of applications. Furthermore, these materials can be readily processed using a variety of manufacturing methods, including additive manufacturing.

A magnesium (Mg) alloy used may preferably be a Mg-Y-RE-Zr 25 alloy, particularly preferably a Mg-Y-Nd-Zr alloy, which is also known as a WE43 alloy. The rare earth (RE) elements Dy, Y, Nd and Gd may have a low toxicity when used as alloying elements in magnesium alloys and exhibit advantageous mechanical and corrosion properties.

A magnesium (Mg) alloy used can also be an Mg-Y-Nd alloy with or without the addition of Zr. In a preferred embodiment, such a magnesium alloy has an yttrium content between 3 wt.% and 5 wt.% and an Nd content between 2 wt.% and 4 wt.%.

A magnesium (Mg) alloy used can also comprise calcium and zinc, preferably as an Mg-Ca-Zn alloy or as an Mg-Zn-Ca alloy, each with or without the addition of Zr. The Ca and Zn contents can preferably each be less than 1 wt.% or less than 2 wt.% or less than 5 wt.%.

Regarding physical materialization of a structure according to the invention, both rapid prototyping routes and industrial mass production and massive customization options are available. For the rapid prototyping route, a huge variety of polymeric filaments with different degradation rates are available and can be printed with multi-extrusion fused deposition modeling (FDM) or fused filament fabrication (FFF) technologies. Their combined use may lead to the affordable creation of complex shape-changing structures triggered by degradation, for example by immersion in water, using a rapid prototyping approach for countless applications. As regards industrialization, both as mass produced structures and as massively customized structures using high performance materials, laser powder bed fusion provides a beneficial option. Employing multi-material laser powder bed fusion, it is feasible to combine materials and in particular alloys with different degradation rates and reach the described multi-material shape-changing structures triggered by selective or differential degradation.

A specific advantage of a structure according to the invention is that it allows for an extensive shape change of the second part, thereby providing a large field of application. In particular, the elastic deformation of the second part may amount to a shape change of at least 100% or at least 200% or at least 300%, i.e. the shape change may cover an alteration of at least one physical dimension of the second part of at least 100% (thus doubling or halving that dimension) or 200% (thus tripling that dimension or reducing to one-third) or 300% (thus quadrupling that dimension or reducing to one-fourth).

### EXEMPLARY EMBODIMENTS

Exemplary embodiments of the invention will be explained below with reference to the figures.

### BRIEF DESCRIPTION OF THE FIGURES

- Fig. 1a:: is a schematic representation of a first embodiment of a structure according to the invention in an initial state.
- Fig. 1b:: is a schematic representation of the structure according to the first embodiment in a final state.
- Fig. 1c:: is a schematic representation showing the evolution of an elastic shape change of a second part of the structure according to the first embodiment.
- Fig. 2a:: is a schematic representation of a second embodiment of a structure according to the invention in an initial state.
- Fig. 2b:: is a schematic representation of the structure according to the second embodiment in a first intermediate state.
- Fig. 2c:: is a schematic representation of the structure according to the second embodiment in a second intermediate state.
- Fig. 2d:: is a schematic representation of the structure according to the second embodiment in a final state.
- Fig. 2e:: is a schematic representation showing the evolution of an elastic shape change of a second part of the structure according to the second embodiment.
- Fig. 3a:: is a schematic representation of a third embodiment of a structure according to the invention in an initial state.
- Fig. 3b:: is a schematic representation of the structure according to the third embodiment in a first intermediate state.
- Fig. 3c:: is a schematic representation of the structure according to the third embodiment in a second intermediate state.
- Fig. 3d:: is a schematic representation of the structure according to the third embodiment in a final state.
- Fig. 3e:: is a schematic representation showing the evolution of an elastic shape change of a second part of the structure according to the third embodiment.
- Fig. 4a:: is a schematic representation of a perspective view of a fourth embodiment of a structure according to the invention in an original state
- Fig. 4b: is a schematic representation of a side view of the structure according to the fourth embodiment in an initial state.
- Fig. 4c:: is a schematic representation of a side view of the structure according to the fourth embodiment in a first intermediate state.
- Fig. 4d:: is a schematic representation of a side view of the structure according to the fourth embodiment in a second intermediate state.
- Fig. 4e:: is a schematic representation of a side view of the structure according to the fourth embodiment in a third intermediate state
- Fig. 4f:: is a schematic representation of a side view of the structure according to the fourth embodiment in the final state.
- Fig. 4g:: is a schematic representation showing the evolution of an elastic shape change of a second part of the structure according to the fourth embodiment.
- Fig. 5a:: is a schematic representation of a perspective view of a fifth embodiment of a structure according to the invention in an original state
- Fig. 5b: is a schematic representation of a perspective view of the structure according to the fifth embodiment in an initial state.
- Fig. 5c:: is a schematic representation of a perspective view of the structure according to the fifth embodiment in a first intermediate state.
- Fig. 5d:: is a schematic representation of a perspective view of the structure according to the fifth embodiment in a second intermediate state.
- Fig. 5e:: is a schematic representation of a perspective view of the structure according to the fifth embodiment in a third intermediate state
- Fig. 5f:: is a schematic representation of a perspective view of the structure according to the fifth embodiment in the final state.

### DETAILED DESCRIPTION OF THE EXEMPLARY EMBODIMENTS

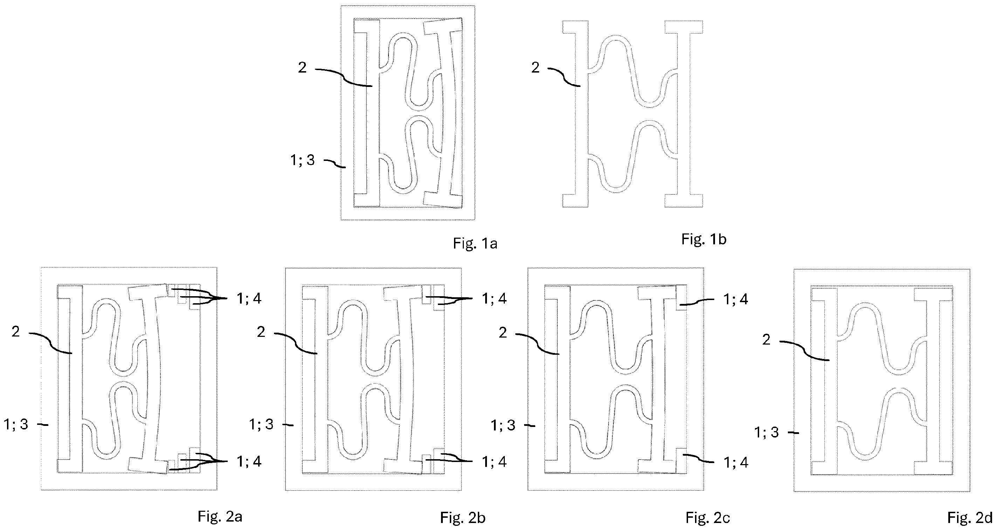

Fig. 1a and 1b show a first embodiment of a structure according to the invention. Fig. 1a shows the structure in an initial state, in which a rapidly degradable interlocking framework as a first part 1 clamps a slowly or non-degradable, elastically deformed second part 2. The first and second parts 1, 2 may be separate elements. In Fig. 1b, the first part 1 has degraded and thereby released the second part 2 which may then freely expand elastically until reaching a final state.

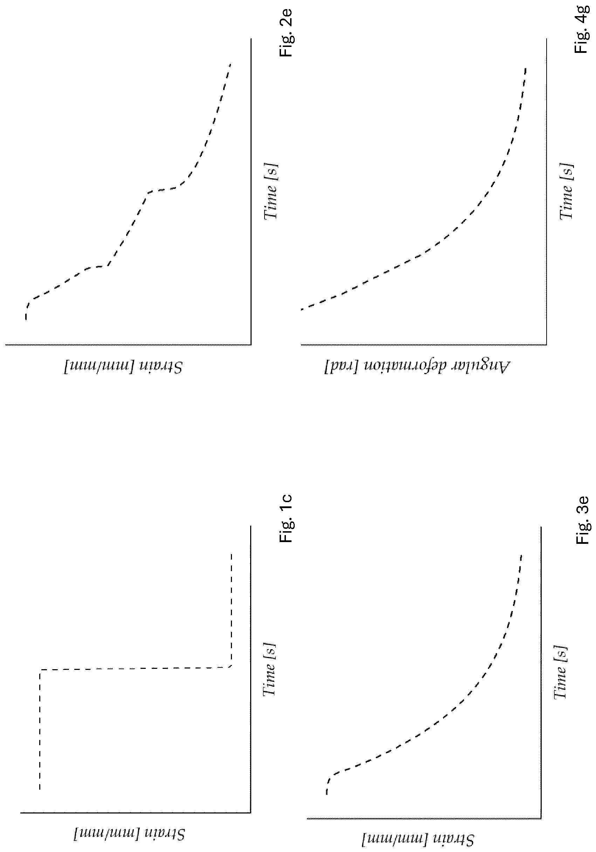

The release of the elastically deformed second part 2 may be spontaneous, e.g. by a catastrophic destruction of the first part 1 after degradation to a specific extent, as it is shown in Fig. 1c.

According to an alternative embodiment (not shown), multiple frameworks representing a first part 1 of a structure according to the invention and degrading at different rates or combining thicknesses within the degradable structure, allow for multi-step release of the elastically deformed second part 2.

Fig. 2a - 2d show a second embodiment of a structure according to the invention. Fig. 2a - 2c show the structure in an initial state (Fig. 2a) and in two intermediate states (Fig. 2b and 2c), in which an interlocking framework 3 and degradable interlocking elements 4 as a first part 1 clamps a slowly or non-degradable, elastically deformed second part 2. The interlocking elements 4 are positioned between the framework 4 and the second part 2. The first and second parts 1, 2 may be separate elements. The interlocking elements 4 degrade sequentially, thereby leading to a multi-step release of the elastically deformed second part 2. In Fig. 2d, the interlocking elements 4 of the first part 1 have degraded totally and thus released the second part 2 to a maximum extent (final state). A respective evolution of the deformation of the second part 2, which combines a stepwise and continuous shape change, is shown in Figure 2e.

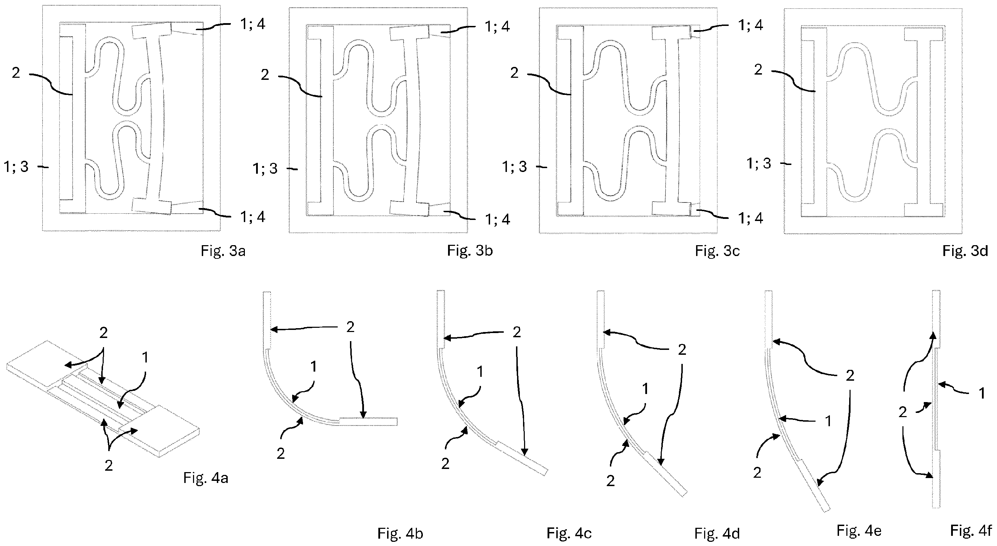

The third embodiment according to Fig. 3a - 3d differs from the embodiment of Figs. 2a - 2d in that an interlocking element 4 is provided, the size of which is reduced constantly due to degradation, thereby leading to a continuous shape change of the second part 2. The interlocking element 4 may have a variable, in particular constantly varying thickness. A respective evolution of the deformation of the second part 2 is shown in Figure 3e.

According to alternative embodiments, degradable interlocking elements 4 may be designed as pins, snap fits or threads. Instead of resorting to an external cage or framework, it is also possible to employ smaller biodegradable regions and derived geometrical interlockings. These may take inspiration from joints typically employed in the industry, like bolts, snap fits or screws.

Single interlocking elements 4 may lead to single-step or eventually progressive actuation, while designs including multiple interlocking elements 4 may allow for multi-stepped actuations. Progressive actuation is possible through special designs of the interlocking elements 4, normally based on gradients of thickness or porosity. In specific cases an interlocking element 4 may be printed as a single degradable element (for example, an external bar or pin that interlocks the shape-changing structure through a designed hole or slot), which would be a simplified version of the degradable framework explained above. However, bi-material and multi-material printing facilitates the simultaneous creation of a structure comprising the slowly or non-degradable second part 2 and the (at least in one section) rapidly degradable first part 1. Hence, a single structure would be obtained.

Fig. 4a - 4e show a fourth embodiment of a structure according to the invention. Fig. 4b - 4d show the structure in an initial state (Fig. 4b) and in three intermediate states (Fig. 4c - 4e), while Fig. 4f shows the structure in a final state. The structure comprises a first section, representing a first part 1 of the structure and made from a rapidly degradable material, and a second section, representing a second part 2 of the structure and made from a slowly or non-degradable material. Due to a (faster) degradation of the first part 1, an elastic shape change of the second part 2 will be released continuously. A respective evolution of the angular deformation of the second part 2, which is a continuous shape change, is shown in Figure 4e.

In the initial state according to Fig. 4b, the first part may be plastically deformed to secure an elastically deformed form of the second part. In order to convert the structure to the initial state, it may be bended starting from an original state as shown in Fig. 4a, whereas the bending leads to the plastic deformation of the first part 1 and to the elastic deformation of the second part 2.

According to an alternative embodiment, an interwoven structure with a rapidly degradable first section being plastically deformed through the interaction with the elastic second section may be provided. Further, degradable interlocking elements 4 that are plastically deformed can be provided, either as separate elements, in the form of coffins, frameworks 3, staples or rivets, or as at least one a rapidly degradable section (first part 1) next to at least one a slowly or non-degradable section, elastic (second part 2) in an integrated element.

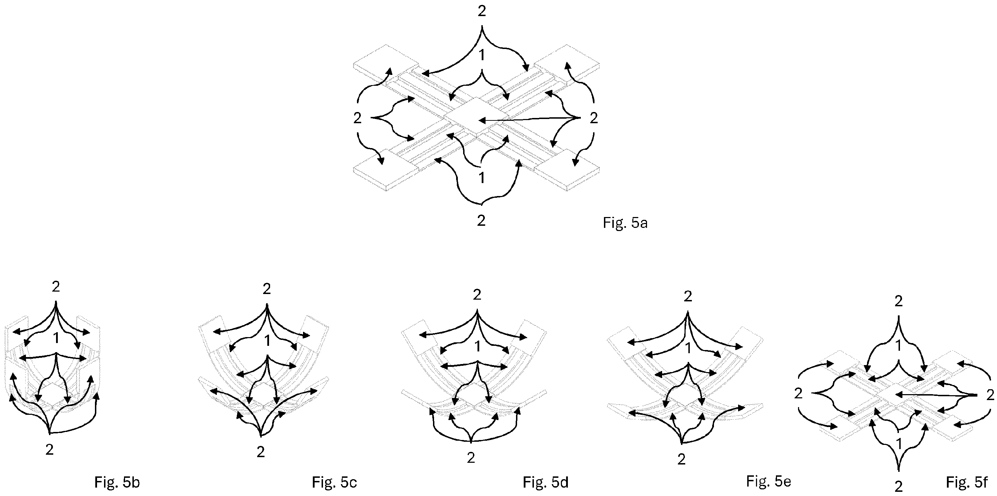

Figs. 5a to 5f show a fifth embodiment of a structure according to the invention, which combines integrally several structures according to Fig. 4a - 4f to provide a blossoming type of structure.

### EXEMPLARY USE CASES

Craniosynostosis, characterized by the premature fusion of the calvarial sutures, can lead to increased intracranial pressure, permanent brain injury, mid-facial hypoplasia, and orbital deformation. Two state-of-the-art therapeutic approaches are currently discussed to cure Craniosynostosis. The first consists of delaying the surgery up to an age of 9 - 12 months and carrying out open fronto-orbital advancement and bone remodeling. This ensures optimal results but at the cost of a largely invasive intervention. The second is an early intervention (at 3 months) involving an endoscopic suturectomy and letting the brain grow and expand the cranial vault. This approach, however, does not guarantee control over the growth rate and shape and symmetry of the final results. The use of a structure according to the invention may avoid these problems and minimize risks associated with large open surgeries; it could guide calvarial growth via a minimally invasive implantation at an early age. After virtual diagnosis and surgery planning, osteotomies would be made locally, and patient-specific realignment (e.g., antero-posterior advancement and transverse expansion of 8-15 mm depending on the severity and the type of deformity) would occur through the controlled expansion of the structure. After repositioning, the degradation of the implanted structure would promote bone growth and subsequent fusion between the fragments. In addition to improving the lives of very young patients, this radical treatment approach is expected to reduce healthcare costs and be a commercially viable product.

Skin expansion is usually required during the reconstruction of large and disfiguring defects or malformations, e.g., the complete loss of the nose or giant nevi and allows for plastic reconstruction with neighboring skin of similar color, texture, sensation, thickness, and hair-bearing capability, while sparing donor site morbidity and maintaining nerve sensibility. Current skin expanders, which are essentially implanted rubber balloons, are inflated with weekly saline injections via a subcutaneous port, requiring regular outpatient visits with painful punctures and the associated risk of expander infection. Inflation driven by the controlled degradation of a self-expanding structure according to the invention, in particular in form of a lattice, could radically change the key points of expander therapy with respect to invasiveness, patient selection and tolerance, as well as financial and personnel burden. Injections would become obsolete, widening the field of application to more fragile patients, such as children, and encouraging therapy adherence. The proposed structure would also be compatible with incorporation of novel molecular therapies in order to encourage tissue growth and blood vessel supply. Like the craniosynostosis device, an autonomous skin expander would improve patient quality of life and reduce healthcare costs.

Other useful applications of a structure according to the invention comprise surgical tools or endoluminal devices such as: biliary stent or graft; urethra stent or graft; ureter stent or graft; oesophageal stent or graft; vascular graft or stent; in particular a coronary or small diameter stent or graft; duodenal stent or graft; renal stent or graft; hepatic stent or graft; venous stent or graft; brain stent or graft, in particularly flow diverter; nerve graft or conduit; tendon and ligament graft or conduit; suture-less anastomosis device; periosteal replacement.

### LIST OF REFERENCE SIGNS

- 1: first part
- 2: second part
- 3: framework
- 4: interlocking element

### CITED REFERENCES

1. Ahmed, A., Arya, S., Gupta, V., Furukawa, H. & Khosla, A. 4D printing: Fundamentals, materials, applications and challenges. Polymer 228, 123926 (2021)
2. Choi, J., Kwon, O.-C., Jo, W., Lee, H. J. & Moon, M.-W. 4D Printing Technology: A Review. 3D Printing and Additive Manufacturing 2, 159-167 (2015)
3. Tibbits, S. Skylar Tibbits: The emergence of '4D printing' | TED Talk. https://www.ted.com/talks/skylar_tibbits_the_emergence_of_4d_printing
4. Sahafnejad-Mohammadi, I., Karamimoghadam, M., Zolfagharian, A., Akrami, M. & Bodaghi, M. 4D printing technology in medical engineering: a narrative review. J Braz. Soc. Mech. Sci. Eng. 44, 233 (2022)
5. Agarwal, T. et al. 4D printing in biomedical applications: emerging trends and technologies. J. Mater. Chem. B 9, 7608-7632 (2021)
6. Khalid, M. Y. et al. 4D printing: Technological developments in robotics applications. Sensors and Actuators A: Physical 343, 113670 (2022)
7. de Marco, C., Pané, S. & Nelson, B. J. 4D printing and robotics. Science Robotics 3, eaau0449 (2018)
8. Mitchell, A., Lafont, U., Ho 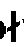 yńska, M. & Semprimoschnig, C. Additive manufacturing - A review of 4D printing and future applications. Additive Manufacturing 24, 606-626 (2018)
9. Tibbits, S., McKnelly, C., Olguin, C., Dikovsky, D. & Hirsch, S. 4D Printing and Universal Transformation. in 539-548 (2014) (doi: 10.52842/conf.acadia.2014.539)
10. Diaz Lantada, A. Systematic Development Strategy for Smart Devices Based on Shape-Memory Polymers. Polymers 9, 496 (2017)
11. Akbari, S., Sakhaei, A. H., Panjwani, S., Kowsari, K. & Ge, Q. 14 - Shape-reversible 4D printing aided by shape memory alloys. in Smart Materials in Additive Manufacturing (eds. Bodaghi, M. & Zolfagharian, A.) 387-406 (Elsevier, 2022). doi:10.1016/B978-0-323-95430-3.00014-2
12. Wang, F. et al. 4D printing of ceramic structures. Additive Manufacturing 63, 103411 (2023)
13. Abdullah, T. & Okay, O. 4D Printing of Body Temperature-Responsive Hydrogels Based on Poly(acrylic acid) with Shape-Memory and Self-Healing Abilities. ACS Appl. Bio Mater. 6, 703-711 (2023)
14. Spiegel, C. A., Hackner, M., Bothe, V. P., Spatz, J. P. & Blasco, E. 4D Printing of Shape Memory Polymers: From Macro to Micro. Advanced Functional Materials 32, 2110580 (2022)
15. Micalizzi, S., Diaz Lantada, A. & De Maria, C. Shape-memory actuators manufactured by dual extrusion multimaterial 3D printing of conductive and nonconductive filaments. Smart Mater. Struct. 28, 105025 (2019)
16. Luo, B., Zhu, Z., Xu, X. & Bian, C. 4 - 4D-printed low-voltage electroactive polymers modeling and fabrication. in Smart Materials in Additive Manufacturing (eds. Bodaghi, M. & Zolfagharian, A.) 107-150 (Elsevier, 2022) (doi:10.1016/B978-0-12-824082-3.00029-5)
17. Gastaldi, M. et al. 4D printing of light activated shape memory polymers with organic dyes. Mol. Syst. Des. Eng. 8, 323-329 (2023)
18. Jeong, H. Y., Woo, B. H., Kim, N. & Jun, Y. C. Multicolor 4D printing of shape-memory polymers for light-induced selective heating and remote actuation. Sci Rep 10, 6258 (2020)
19. 4D Printing of Humidity-Driven Seed Inspired Soft Robots - Cecchini - 2023 - Advanced Science - Wiley Online Library (https://onlinelibrary.wiley.com/doi/10.1002/advs.202205146)
20. de Kergariou, C., Demoly, F., Perriman, A., Le Duigou, A. & Scarpa, F. The Design of 4D-Printed Hygromorphs: State-of-the-Art and Future Challenges. Advanced Functional Materials 33, 2210353 (2023)
21. Lantada, A. D. et al. Combining smart materials for enhancing intelligent systems: initial studies, success cases and research trends. Smart Structures and Systems, An International Journal 14, 517-539 (2014)
22. Ma, S. et al. Recent progress in 4D printing of stimuli-responsive polymeric materials. Sci. China Technol. Sci. 63, 532-544 (2020)
23. Chen, D. et al. 4D Printing Strain Self-Sensing and Temperature Self-Sensing Integrated Sensor-Actuator with Bioinspired Gradient Gaps. Advanced Science 7, 2000584 (2020)
24. Wang, Y. & Li, X. 4D printing reversible actuator with strain self-sensing function via structural design. Composites Part B: Engineering 211, 108644 (2021)
25. Wu, J. et al. Multi-shape active composites by 3D printing of digital shape memory polymers. Sci Rep 6, 24224 (2016)
26. Zhang, Y. et al. Water-responsive 4D printing based on self-assembly of hydrophobic protein "Zein" for the control of degradation rate and drug release. Bioactive Materials 23, 343-352 (2023)
27. Sun, W. et al. Biodegradable, Sustainable Hydrogel Actuators with Shape and Stiffness Morphing Capabilities via Embedded 3D Printing. Advanced Functional Materials n/a, 2303659
28. Hartmann, F., Baumgartner, M. & Kaltenbrunner, M. Becoming Sustainable, The New Frontier in Soft Robotics. Advanced Materials 33, 2004413 (2021)
29. CN 117 752 473 A
30. Li, M. et al. Microstructure, mechanical properties, corrosion resistance and cytocompatibility of WE43 Mg alloy scaffolds fabricated by laser powder bed fusion for biomedical applications. Materials Science and Engineering: C 119, 111623 (2021)
31. CN 108 577 954 A
32. Magnesium and its alloys as orthopedic biomaterials: A review - ScienceDirect (https://www.sciencedirect.com/science/article/abs/pii/S0142961205009014)
33. Staiger, M. P., Pietak, A. M., Huadmai, J. & Dias, G. Magnesium and its alloys as orthopedic biomaterials: A review. Biomaterials 27, 1728-1734 (2006)
34. Patterson, J. et al. Hyaluronic acid hydrogels with controlled degradation properties for oriented bone regeneration. Biomaterials 31, 6772-6781 (2010)
35. Lantada, A. D. et al. Additive Manufacturing of Nitinol for Smart Personalized Medical Devices: Current Capabilities and Challenges. in vol. 2 123-134 (SCITEPRESS, 2024)
36. Molina Aldareguia, J. et al. Impresión 4D empleando polimeros y aleaciones con memoria de forma para una nueva generación de dispositivos médicos inteligentes. (2022)
37. Ge, Q., Dunn, C. K., Qi, H. J. & Dunn, M. L. Active origami by 4D printing. Smart Mater. Struct. 23, 094007 (2014)
38. Lu, Q. et al. Degrade to Function: Towards Eco-friendly Morphing Devices that Function Through Programmed Sequential Degradation. Preprint at https://doi.org/10.48550/arXiv.2408.01660 (2024)

## Claims

1. A structure comprising a first part (1) made at least partially from a rapidly degradable material and a second part (2) made at least partially from a slowly or non-degradable material, whereas the second part (2) has an elastically deformed form, which is secured by the first part (1) such that an at least partial degradation of the first part (1) will release an elastic shape change of the second part (2).

2. The structure according to claim 1, **characterized in that** the first part (1) is made from a rapidly biodegradable material and/or the second part (2) is made from a slowly or non-biodegradable material.

3. The structure according to claim 2, **characterized in that** the second part (2) is secured by the first part (1) such that a progressing degradation of the first part (1) leads to a progressing shape change of the second part (2).

4. The structure according to claim 3, **characterized in that** the progressing shape change of the second part (2) is stepwise or continuous.

5. The structure according to one of the preceding claims, **characterized in that** the first part (1) and the second part (2) are integral sections of an integrated element.

6. The structure according to one of the claims 1 to 4, **characterized in that** the first part (1) and the second part (2) are separate elements.

7. The structure according to one of the preceding claims, **characterized in that** the parts (1, 2) each comprise at least one interlocking element (4), whereas those interlocking elements (4) are interacting to create a connection of the parts (1, 2) which secures the second part (2) in the elastically deformed form, and whereas a degradation of the at least one interlocking element (4) of the first part (1) leads to a release of the connection.

8. The structure according to one of the preceding claims, **characterized in that** the first part (1) is plastically deformed when the second part (2) has the elastically deformed form.

9. The structure according to one of the preceding claims, **characterized in that** the first part (1) and/or the second part (2) is/are produced by additive manufacturing.

10. The structure according to one of the preceding claims, **characterized in that** the rapidly degradable material and/or the slowly degradable material is or comprises at least a metal and/or a polymer and/or a ceramic.

11. The structure according to one of the preceding claims, **characterized in that**
• the rapidly degradable material is or comprises at least one of: Mg, Zn, Ti, Ni, PVA, PCL, hydroxyapatite
and/or
• the slowly or non-degradable material is or comprises at least one of: Zn, Fe, Mo, Ti, Ni, PET, PETG, PLA, ABS, tricalcium phosphate, alumina or zirconia.

12. The structure according to one of the preceding claims, **characterized in that** the elastic deformation of the second part (2) amounts to a shape change of at least 100% or at least 200% or at least 300%.

13. The use of a structure according to one of the preceding claims as a bone distraction actuator and/or as a skin expansion device and/or as a tissue engineering scaffold and/or as a surgical tool and/or as an endoluminal device.
